# EUROPÄISCHE PATENTSCHRIFT

(11) **EP 3 267 973 B1**
(45) Veröffentlichungstag und Bekanntmachung des Hinweises auf die Patenterteilung: **09.10.2019**
(21) Anmeldenummer: 16701524.7
(22) Anmeldetag: 26.01.2016
(51) Int. Cl.: A61K 8/81, A61Q 5/06

(54) **MITTEL UND VERFAHREN ZUR TEMPORÄREN VERFORMUNG KERATINHALTIGER FASERN**
PRODUCT AND METHOD FOR THE TEMPORARY SHAPING OF KERATIN-CONTAINING FIBERS
PRODUIT ET PROCÉDÉS DE MISE EN EN FORME TEMPORAIRE DE FIBRES KÉRATINIQUES

(30) Priorität: 09.03.2015 DE 102015204152
(43) Veröffentlichungstag der Anmeldung: 17.01.2018
(73) Patentinhaber: Henkel AG & Co. KGaA, 40589 Düsseldorf (DE)
(72) Erfinder: LANGE, Julia Bibiane, 24576 Bad Bramstedt (DE); PULS, Anna, 21423 Winsen (Luhe) (DE); MARTINEZ, Cyrielle, 22765 Hamburg (DE); RICHTERS, Bernd, 21129 Hamburg (DE)
(86) Internationale Anmeldenummer: PCT/EP2016/051566
(87) Internationale Veröffentlichungsnummer: WO 2016/142092

(56) Entgegenhaltungen:
- WO-A1-2011/057882
- WO-A1-2012/072774
- WO-A2-2014/095163
- ANONYMOUS: "Ashland brings performance and style to crystal clear gel with AquaStyle", INTERNET CITATION, April 2014 (2014-04), Seiten 1-2, XP002751471, Gefunden im Internet: URL:http://files.shareholder.com/downloads /ASH/0x0x739330/f61f8ac2-932b-4e99-afa7-0d 1962337028/ASH_News_2014_4_1_Products_and_ Services.pdf [gefunden am 2015-11-26]
- Anonymous: "Ashland brings performance and style to crystal clear gel with AquaStyle(TM) (NYSE:ASH)", , 1. April 2014 (2014-04-01), XP55205095, Gefunden im Internet: URL:http://investor.ashland.com/releasedet ail.cfm?releaseid=836949 [gefunden am 2015-07-28]

## Beschreibung

Die vorliegende Erfindung betrifft die Verwendung einer kosmetischen Zusammensetzung zur Haarfestigung bzw. zur temporären Umformung von keratinischen Fasern, insbesondere menschlichen Haaren, wobei die Zusammensetzung eine Kombination von zwei anionischen Acrylatpolymeren enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden. Handelt es sich bei dem Stylingmittel um ein Gel oder eine Paste, sollen die Polymere zudem verdickende Eigenschaften besitzen.

Bekannte anionische Polymere, die in Haarfestigungsprodukten Anwendung finden, sind Acrylatcopolymere mit zwei oder mehr Struktureinheiten. Bestimmte derartige Terpolymere, welche durch Emulsionspolymerisation der Monomere n-Butylmethacrylat, Methacrylsäure, und Ethylacrylat erhalten werden, sind in der internationalen Anmeldung WO 2012/072774 A1 beschrieben. Die internationale Anmeldung WO 2014/095163 A1 beschreibt die Verwendung entsprechender Terpolymere in Haarschäumen zur Verbesserung des Haarvolumens.

Weiterhin sind hydrophob modifizierte Acrylatcopolymere (INCI: Acrylates Copolymer (and) Water) im Handel erhältlich, die im Wesentlichen als Verdickungsmittel wirken. Das Datenblatt AquaStyle® SH-100 Polymer (Ashland Inc.) beschreibt ein solches Acrylatcopolymer und dessen Verwendung in Kombination mit Carbomeren. Es wird eine Eignung für kristallklare Haargele, eine gute Anfangssteifheit, Feuchtigkeitsbeständigkeit und eine Langzeitwirkung beschrieben.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden - müsste und sich zudem für die Herstellung stabil viskoser sowie stabil transparenter kosmetischer Zusammensetzungen eignen. Insbesondere sind derzeit erhältliche Stylingmittel noch dahingehend verbesserbar, dass eine gute Kombination aus Steifheit (Stiffness) und Langzeithalt (High Humidity Curl Retention) nicht immer ausreichend gewährleistet wird. Es ist daher eine Aufgabe der vorliegenden Erfindung, derartige Stylingmittel "bereitzustellen, die neben den oben genannten Eigenschaften insbesondere sowohl eine gute Steifheit als auch einen guten Langzeithalt ergeben.

Dies wurde erfindungsgemäß durch eine Kombination zweier bestimmter, voneinander verschiedener anionischer Acrylatpolymere erreicht.

Durch die vorliegende Erfindung wird bereitgestellt:
1. Verwendung einer kosmetischen Zusammensetzung zur temporären Umformung keratinischer Fasern, welche enthält:
   (a) mindestens ein Acrylatcopolymer (a), welches ausschließlich aus folgenden Monomereinheiten aufgebaut ist:
      (a1) n-Butylmethacrylat,
      (a2) Methacrylsäure,
      (a3) Ethylacrylat,
      und
   (b) mindestens ein anionisches Acrylatcopolymer (b), welches von dem Acrylatcopolymer (a) verschieden ist und nur aus den Monomereinheiten (b1), (b2) und (b3) aufgebaut ist, wobei:
      (b1) mindestens eine (Meth)Acrylsäureeinheit, welche Methacrylsäure ist,
      (b2) mindestens eine (Meth)Acrylsäureethylestereinheit, welche Ethylacrylat ist, und
      (b3) mindestens eine (Meth)Acrylsäureestereinheit, die von der (Meth)Acrylsäureethylestereinheit (b2) verschieden ist und welche ein (Meth)Acrylsäurealkylester mit einer linearen oder verzweigten Alkylgruppe mit 2 bis 12 Kohlenstoffatomen ist, aufweist,
      zur Verbesserung der Feuchtebeständigkeit temporär verformter keratinischer Fasern.
2. Verwendung nach Punkt 1, wobei das mindestens eine Acrylatcopolymer (a) bezogen auf das Gewicht des Copolymers (a) 60 bis 80 Gew.-% n-Butylmethacrylat, 10 bis 30 Gew.-% n-Methacrylsäure und 5 bis 15 Gew.-% Ethylacrylat aufweist.
3. Verwendung nach einem der vorhergehenden Punkte, wobei das Copolymer (a) durch Emulsionspolymerisation hergestellt ist.
4. Verwendung nach einem der vorhergehenden Punkte, wobei die Zusammensetzung das Copolymer (a) in einem Anteil von 0,2 bis 4,0 Gew.-%, bevorzugt 1,0 bis 3,5 Gew.-% und insbesondere von 1,5 bis 3,0 Gew.-% enthält, bezogen auf das Gesamtgewicht der kosmetischen Zusammensetzung.
5. Verwendung nach einem der vorhergehenden Punkte, wobei die Zusammensetzung das anionische Acrylatcopolymer (b) in einem Anteil von 0,1 bis 5,0 Gew.-%, bevorzugt 1,0 bis 4,0 Gew.-% und insbesondere von 1,5 bis 3,0 Gew.-% enthält, bezogen auf das Gesamtgewicht der kosmetischen Zusammensetzung.
6. Verwendung nach einem der vorhergehenden Punkte, wobei das anionische Acrylatcopolymer (b) bei einem Feststoffgehalt von 2 Gew.-% in einer wässerigen neutralisierten Lösung bei 25°C eine Viskosität von 60000 bis 120000 cPs aufweist.
7. Verwendung nach einem der vorhergehenden Punkte, wobei das anionische Acrylatcopolymer (a) eines mit der INCI-Bezeichnung Acrylates Copolymer, insbesondere Tilamar® Fix (DSM), ist.
8. Verwendung nach einem der vorhergehenden Punkte, wobei das anionische Acrylatcopolymer (b) eines mit der INCI-Bezeichnung Acrylates Copolyme (and) Water, insbesondere AquaStyle SH-100 (Ashland Inc.), ist.
9. Verwendung nach einem der vorhergehenden Punkte, wobei das anionische Acrylatcopolymer (a) eines mit der INCI-Bezeichnung Acrylates Copolymer und das anionische Acrylatcopolymer (b) eines mit der INCI-Bezeichnung Acrylates Copolymer (and) Water ist.
10. Verwendung nach einem der vorhergehenden Punkte, wobei das anionische Acrylatcopolymer (a) Tilamar® Fix (DSM) ist und das anionische Acrylatcopolymer (b) AquaStyle® SH-100 (Ashland Inc.) ist.
11. Verwendung nach einem der vorhergehenden Punkte, welche, bezogen auf das Gesamtgewicht der kosmetischen Zusammensetzung, enthält:
   0,5 bis 10 Gew-% des anionischen Acrylatcopolymers (a), und
   0,1 bis 15 Gew.-% des anionischen Acrylatcopolymers (b).
12. Verwendung nach einem der vorhergehenden Punkte, enthaltend, bezogen auf das Gesamtgewicht der kosmetischen Zusammensetzung:
   4,0 bis 8,0 Gew.-% des anionischen Acrylatcopolymers (a), und
   5,0 bis 10 Gew.-% des anionischen Acrylatcopolymers (b).
13. Verwendung einem der vorhergehenden Punkte, wobei die Zusammensetzung weiterhin mindestens ein von den Acrylatcopolymeren (a) und (b) verschiedenes Polymer (c), insbesondere ein anionisches oder nichtionisches Polymer (c), enthält.
14. Verwendung nach einem der vorherigen Punkte, dadurch gekennzeichnet, dass es bezogen auf sein Gesamtgewicht weiterhin
   c) 1,0 bis 10 Gew.-% Polyvinylpyrrolidon und/oder Vinylpyrrolidon/Vinylacetat-Copolymer, vorzugsweise Polyvinylpyrrolidon enthält.
15. Verwendung nach Punkt 16, dadurch gekennzeichnet, dass der Gewichtsanteil des Polyvinylpyrrolidons und/oder Vinylpyrrolidon/Vinylacetat-Copolymers c) am Gesamtgewicht der kosmetische Zusammensetzung 2,0 bis 8,5 Gew.-%, vorzugsweise 3,0 bis 7,0 Gew.-% beträgt.
16. Verwendung nach einem der vorhergehenden Punkte, wobei die Zusammensetzung Wasser in einem Anteil von 50 und 95 Gew.-%, bevorzugt zwischen 60 und 90 Gew.-% und insbesondere zwischen 65 und 85 Gew.-%enthält, bezogen auf das Gesamtgewicht der kosmetischen Zusammensetzung.
17. Verwendung nach einem der vorhergehenden Punkte, wobei die Zusammensetzung als Haargel, Haarspray, Haarschaum oder Haarwachs, insbesondere als Haargel, vorliegt.

Es wurde im Rahmen der vorliegenden Erfindung überraschend festgestellt, dass durch Kombination zweier an sich bekannter Bestandteile, die in Stylingprodukten bereits Anwendung finden, eine verbesserte Feuchtebeständigkeit von Stylingprodukten erhalten werden kann. Andere üblicherweise geforderte Eigenschaften von Stylingprodukten wie Langzeithalt, Steifheit und geringe Klebrigkeit blieben dabei erhalten. Eine derartige gute Kombination von Eigenschaften war selbst bei Kenntnis der Einzelkomponenten nicht zu erwarten und war überraschend. Es zeigte sich experimentell, dass durch die Kombination der beiden Komponenten ein stark überadditiver, also synergistischer Effekt hinsichtlich der Feuchtebeständigkeit erhalten wurde, was sich im HHRC-Test (High Humidity Curl Retention-Test) manifestierte.

Der Begriff keratinische Fasern umfasst erfindungsgemäß Pelze, Wolle und Federn, insbesondere aber menschliche Haare.

Die wesentlichen Bestandteile der erfindungsgemäß verwendeten kosmetischen Zusammensetzung sind das anionisches Acrylatcopolymer (a) und das von dem Acrylatcopolymer (a) verschiedene anionische Acrylatcopolymer (b).

Die erfindungsgemäß eingesetzten Copolymere (a) werden durch Polymerisation, insbesondere durch Emulsionspolymerisation, der Monomere n-Butylmethacrylat, Methacrylsäure und Ethylacrylat erhalten.

In einer bevorzugten Ausführungsform weisen die Copolymere (a) bezogen auf ihr Gewicht 60 bis 80 Gew.-% n-Butylmethacrylat, 10 bis 30 Gew.-% Methacrylsäure und 5 bis 15 Gew.-% Ethylacrylat auf, wobei Copolymere (a) mit einem Gewichtsanteil der Monomere von 65 bis 75 Gew.-% n-Butylmethacrylat, 15 bis 25 Gew.-% Methacrylsäure und 8 bis 12 Gew.-% Ethylacrylat besonders bevorzugt sind. Ganz besonders bevorzugte Copolymere (a) weisen einen Gewichtsanteil der Monomere von 67 bis 72 Gew.-% n-Butylmethacrylat, 18 bis 23 Gew.-% Methacrylsäure, 9 bis 11 Gew.-% Ethylacrylat auf.

Die Copolymere A weisen vorzugsweise ein Molekulargewicht von größer als etwa 100kDa, bevorzugt etwa 130-160 kDa, auf. Die Glasübergangstemperatur beträgt vorzugsweise zwischen 80 und 120°C, insbesondere zwischen 85 bis 105°C, weiter bevorzugt etwa 90 bis 102 °C. Die zuvor beschriebenen Terpolymere als Copolymer (a) werden beispielsweise unter der Bezeichnung Tilamar® Fix A140 (INCI: Acrylates Copolymer; CAS Nummer: 26715-43-5) von der Firma DSM vertrieben.

Die erfindungsgemäß verwendeten kosmetischen Zusammensetzungen enthalten als zweiten wesentlichen Bestandteil ein anionisches Acrylatcopolymer (b).

Das anionische Acrylatcopolymer (b) ist nur aus folgenden Monomereinheiten aufgebaut: mindestens einer (Meth)Acrylsäureeinheit (b1), welche Methacrylsäure aufweist, mindestens einer (Meth)Acrylsäureethylestereinheit (b2), Ethylacrylat aufweist, und mindestens einer (Meth)Acrylsäureestereinheit (b3), die von der (Meth)Acrylsäureethylestereinheit (b2) verschieden ist und welche ein (Meth)Acrylsäurealkylester mit einer linearen oder verzweigten Alkylgruppe mit 2 bis 12 Kohlenstoffatomen ist..

Die mindestens eine (Meth)Acrylsäureestereinheit (b3) ist eine (Meth)Acrylsäurealkylestereinheit. Die Alkylgruppe der (Meth)Acrylsäurealkylestereinheit dient zur Steuerung der Hydrophobizität des Copolymers. Die Alkylgruppe ist eine lineare oder verzweigte Alkylgruppe mit 2 bis 12 Kohlenstoffatomen. Die hydrophobe Gruppe kann erfindungsgemäß auch eine andere hydrophobe als eine Alkylgruppe sein, z. B. eine aromatische Kohlenwasserstoffestergruppe. Als Beispiel sei eine substituierte oder unsubstituierte Phenylestergruppe oder substituierte oder unsubstituierte Alkylenphenylestergruppe genannt, z. B. eine Benzylestergruppe.

Die Viskosität des in der kosmetischen Zusammensetzung verwendeten anionischen Acrylatcopolymers (b) bei einem Feststoffgehalt von 2 Gew.-% und neutralisierter Lösung bei 25°C ist bevorzugt höchstens 60000 bis 120000 cPS.

Geeignete anionische Acrylatcopolymere (b) sind im Handel unter der INCI-Bezeichnung Acrylates Copolymer (and) Water erhältlich. Am bevorzugtesten ist das anionische Acrylatcopolymer (b) AquaStyle® SH-100 Polymer von Ashland, Inc. Dieses hat in der im Handel erhältlichen Form einen Feststoffgehalt von etwa 28 bis 32 Gew.-% und einen pH-Wert von 2,1 bis 4,0.

Die kosmetische Zusammensetzung der vorliegenden Erfindung enthält das Acrylatcopolymer (a) und Acrylatcopolymer (b) in für Stylingmittel üblichen und geeigneten Mengen, die für die spezielle Anwendung und Konfektionierung angepasst werden können.

Die erfindungsgemäß verwendete Zusammensetzung kann das Copolymer (a) beispielsweise in einer Menge von 0,5 bis 10 Gew.-%, bezogen auf das Gesamtgewicht der erfindungsgemäßen Zusammensetzung, enthalten. Bevorzugter sind Anteile des Copolymer (a) von 2,0 bis 9,0 Gew.-% und insbesondere von 4,0 bis 8,0 Gew.-%, jeweils angegeben als Feststoffgehalt aktiver Substanz in der kosmetischen Zusammensetzung.

Die erfindungsgemäß verwendete kosmetische Zusammensetzung enthält das Acrylatcopolymer (b), bezogen auf das Gesamtgewicht der kosmetischen Zusammensetzung, z. B. in einer Menge von 0,1 bis 5,0 Gew.-%, bevorzugt 1,0 bis 4,0 Gew.-%, weiterhin bevorzugt 1,5 bis 3,0 Gew.-%, jeweils angegeben als Feststoffgehalt aktiver Substanz in der kosmetischen Zusammensetzung.

Die erfindungsgemäß eingesetzten kosmetischen Zusammensetzungen zeichnen sich gegenüber alternativen kosmetischen Mitteln neben den oben genannten Vorteilen insbesondere auch durch einen verbesserten Langzeithalt aus. Als für die kosmetischen-Eigenschaften der erfindungsgemäß verwendeten Mittel besonders vorteilhaft hat sich ein Gewichtsverhältnis der Polymere a) und b) in dem kosmetischen 5:1 bis 1:5, vorzugsweise 3:1 bis 1:3 und insbesondere 2:1 bis 1:2 erwiesen.

In einer besonders bevorzugten Ausführungsform der vorliegenden Erfindung enthält die kosmetische Zusammensetzung als das anionische Acrylatcopolymer (a) das im Handel unter der Bezeichnung Tilamar® Fix erhältliche Copolymer und als das anionische Acrylatcopolymer (b) das im Handel unter der Bezeichnung AquaStyle® SH-100 erhältliche Copolymer. Bei dieser Kombination wurden besonders gute Ergebnisse hinsichtlich einer Kombination von Steifheit und Langzeithalt erzielt. Besonders vorteilhaft ist diese Polymerkombination bei Stylingprodukten in Gelform. Weitere allgemein geforderte Eigenschaften von Stylingprodukten, wie z. B. Feuchtebeständigkeit und niedrige Klebrigkeit, werden insbesondere mit dieser Kombination gleichfalls erzielt, besonders bei Konfektionierung als Haargel.

Die Acrylatcopolymere (a) und (b) werden in der kosmetischen Zusammensetzung vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugt eingesetzte kosmetische Mittel enthalten daher 2-Amino-2-methylpropanol. Das 2-Amino-2-methylpropanol wird in den erfindungsgemäß eingesetzten Mitteln vorzugsweise in einer Menge eingesetzt, welche die zur Neutralisation der Acrylatcopolymere (a) und (b) benötigte Menge nicht überschreitet. Vorzugsweise beträgt die in den erfindungsgemäß eingesetzten Zusammensetzungen eingesetzte Mengen an 2-Amino-2-methylpropanol 80 bis 100%, besonders bevorzugt 90 bis 100% und insbesondere 95 bis 100% der zur vollständigen Neutralisation der Acrylatcopolymere (a) und (b) benötigten Menge. In einer bevorzugten Ausführungsform beträgt der Gewichtsanteil des 2-Amino-2-methylpropanols am Gesamtgewicht des kosmetischen Mittels 0,05 bis 7,0 Gew.-%, bevorzugt 0,1 bis 5,0 Gew.% und insbesondere 0,1 bis 3,0 Gew.-%.

Zusammenfassend enthält eine bevorzugte kosmetische Zusammensetzung zur temporären Umformung keratinischer Fasern bezogen auf ihr Gesamtgewicht:
(a) 0,2 bis 4,0 mindestens eines Acrylatcopolymers (a), welches zumindest aus folgenden Monomereinheiten aufgebaut ist:
   (a1) n-Butylmethacrylat,
   (a2) Methacrylsäure,
   (a3) Ethylacrylat, und
   und
(b) 0,1 bis 5,0 mindestens eines anionischen Acrylatcopolymers (b), welches zumindest aus folgenden Monomereinheiten aufgebaut ist:
   (b1) mindestens eine Methacrylsäureeinheit
   (b2) mindestens eine Acrylsäureethylestereinheit
   (b3) mindestens eine Methacrylsäureestereinheit, die von der Acrylsäureethylestereinheit (b2) verschieden ist und eine hydrophobe Gruppe als Estergruppe aufweist.

Bevorzugt enthält die kosmetische Zusammensetzung der vorliegenden Erfindung eine oder mehrere weitere, als Verdickungsmittel oder Gelbildner wirkende Komponente(n), die von dem Acrylatcopolymer (a) und (b) verschieden ist/sind und ebenfalls die Filmbildung unterstützen. Beispiele sind kationische, anionische, nichtionische oder amphotere Polymere. Der Gewichtsanteil dieser weiteren Komponente am Gesamtgewicht der kosmetischen Zusammensetzung kann aufgrund der Anwesenheit der Komponenten (a) und (b) vergleichsweise niedrig sein und beträgt beispielsweise 0,02 bis 3 Gew.-%, bevorzugt 0,05 bis 1,5 Gew.-% und noch bevorzugter 0,2 bis 0,8 Gew.-%.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/ltaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Beispiele für nichtionische Polymere sind:
- Vinylpyrrolidon/Vinylester-Copolymere, wie sie beispielsweise unter dem Warenzeichen Luviskol (BASF) vertrieben werden. Luviskol VA 64 und Luviskol VA 73, jeweils Vinylpyrrolidon/VinylacetatCopolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal und Benecel (AQUALON) vertrieben werden.
- Schellack.
- Polyvinylpyrrolidone, wie sie beispielsweise unter der Bezeichnung Luviskol (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Bevorzugt ist die weitere als Gelbildner wirkende Komponente eine Homopolyacrylsäure (INCI: Carbomer), die im Handel unter dem Namen Carbopol® in unterschiedlichen Ausführungen erhältlich ist. Das Carbomer ist bevorzugt in einem Anteil von 0,02 bis 3 Gew.-%, bevorzugt 0,05 bis 1,5 Gew.-% und noch bevorzugter 0,2 bis 0,8 Gew.-%, in Bezug auf das Gesamtgewicht der kosmetischen Zusammensetzung, enthalten.

Aufgrund ihrer kosmetischen Wirkung in Kombination mit den Copolymeren a) und b) erfindungsgemäß bevorzugt eingesetzte filmbildenden Polymere sind insbesondere die Polyvinylpyrrolidone (INCI-Bezeichnung: PVP) sowie die Vinylpyrrolidon/Vinylacetat-Copolymere (INCI-Bezeichnung VP/VA Copolymer), wobei der Gewichtsanteil dieser Polymere vorzugsweise auf Mengen zwischen 1,0 und 10 Gew.-% beschränkt wird. Besonders bevorzugte erfindungsgemäß eingesetzte kosmetische Zusammensetzungen sind daher dadurch gekennzeichnet, dass sie bezogen auf ihr

Gesamtgewicht weiterhin 1,0 bis 10 Gew.-% Polyvinylpyrrolidon und/oder Vinylpyrrolidon/Vinylacetat-Copolymer, vorzugsweise Polyvinylpyrrolidon enthalten. Besonders bevorzugte kosmetische Mittel weisen einen Gewichtsanteil des Polyvinylpyrrolidons und/oder Vinylpyrrolidon/Vinylacetat-Copolymers c) am Gesamtgewicht des kosmetischen Mittels von 2,0 bis 8,5 Gew.-%, vorzugsweise von 3,0 bis 7,0 Gew.-% auf.

Die erfindungsgemäß verwendete kosmetische Zusammensetzung kann weitere übliche Stoffe von Stylingprodukten enthalten. Als weitere geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße verwendete Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin-und/oder Propylenglykol erhöht der Zusatz von Panthenol die Flexibilität des bei Anwendung des Mittels gebildeten Polymerfilms.

Als Pflegestoff können die erfindungsgemäß verwendeten Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäß verwendete kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von 6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in der erfindungsgemäß verwendeten Zusammensetzung bevorzugt Emulgatoren bzw. oberflächenaktive Mittel enthalten. Bevorzugt sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Die erfindungsgemäß eingesetzten kosmetischen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien mit vorzugsweise mindestens 10 Gew.-% Wasser, berechnet auf das Gesamtgewicht des Mittels.

Besonders bevorzugt enthält der kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält. Ganz besonders bevorzugte kosmetische Mittel weisen bezogen auf ihre Gesamtgewicht einen Wasseranteil zwischen 50 und 95 Gew.-%, bevorzugt zwischen 60 und 90 Gew.-% und insbesondere zwischen 65 und 85 Gew.-% auf.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

### Tabellarische Übersicht

Die Zusammensetzung einiger bevorzugt eingesetzter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Misc | add 100 | add 100 | add 100 | add 100 | add 100 |

| | Formel 1a | Formel 2a | Formel 3a | Formel 4a | Formel 5a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Misc | add 100 | add 100 | add 100 | add 100 | add 100 |

| | Formel 1b | Formel 2b | Formel 3b | Formel 4b | Formel 5b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Misc | add 100 | add 100 | add 100 | add 100 | add 100 |

| | Formel 6 | Formel 7 | Formel 8 | Formel 9 | Formel 10 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 6a | Formel 7a | Formel 8a | Formel 9a | Formel 10a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 6b | Formel 7b | Formel 8b | Formel 9b | Formel 10b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 11 | Formel 12 | Formel 13 | Formel 14 | Formel 15 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 11a | Formel 12a | Formel 13a | Formel 14a | Formel 15a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 11b | Formel 12b | Formel 13b | Formel 14b | Formel 15b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 16 | Formel 17 | Formel 18 | Formel 19 | Formel 20 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

| | Formel 16a | Formel 17a | Formel 18a | Formel 19a | Formel 20a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

| | Formel 16b | Formel 17b | Formel 18b | Formel 19b | Formel 20b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

| | Formel 21 | Formel 22 | Formel 23 | Formel 24 | Formel 25 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

| | Formel 21a | Formel 22a | Formel 23a | Formel 24a | Formel 25a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

| | Formel 21b | Formel 22b | Formel 23b | Formel 24b | Formel 25b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

| | Formel 26 | Formel 27 | Formel 28 | Formel 29 | Formel 30 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 26a | Formel 27a | Formel 28a | Formel 29a | Formel 30a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 26b | Formel 27b | Formel 28b | Formel 29b | Formel 30b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 31 | Formel 32 | Formel 33 | Formel 34 | Formel 35 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 31a | Formel 32a | Formel 33a | Formel 34a | Formel 35a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 31b | Formel 32b | Formel 33b | Formel 34b | Formel 35b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 36 | Formel 37 | Formel 38 | Formel 39 | Formel 40 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 36a | Formel 37a | Formel 38a | Formel 39a | Formel 40a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 36b | Formel 37b | Formel 38b | Formel 39b | Formel 40b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 41 | Formel 42 | Formel 43 | Formel 44 | Formel 45 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 41a | Formel 42a | Formel 43a | Formel 44a | Formel 45a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 41b | Formel 42b | Formel 43b | Formel 44b | Formel 45b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 46 | Formel 47 | Formel 48 | Formel 49 | Formel 50 |
|---|---|---|---|---|---|
| Copolymer a) | 0,2 bis 4,0 | 1,0 bis 3,5 | 1,0 bis 3,5 | 1,5 bis 3,0 | 1,5 bis 3,0 |
| Copolymer b) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 46a | Formel 47a | Formel 48a | Formel 49a | Formel 50a |
|---|---|---|---|---|---|
| Copolymer a): Acrylates Copolymer | 0,2 bis 4,0 | 1,0 bis 3,5 | 1,0 bis 3,5 | 1,5 bis 3,0 | 1,5 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 46b | Formel 47b | Formel 48b | Formel 49b | Formel 50b |
|---|---|---|---|---|---|
| Copolymer a): Tilamar® Fix A140 (Angaben als Feststoffgehalt) | 0,2 bis 4,0 | 1,0 bis 3,5 | 1,0 bis 3,5 | 1,5 bis 3,0 | 1,5 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Wasser | 50 bis 95 | 50 bis 95 | 60 bis 90 | 60 bis 90 | 65 bis 85 |
| Misc | add 100 | add 100 | add 100 | add 100 | add 100 |

Unter "Misc" ist erfindungsgemäß ein kosmetischer Träger zu verstehen, insbesondere (sofern nicht separat angeführt) Wasser und ggf. weitere übliche Bestandteile von Stylingprodukten.

Die kosmetische Zusammensetzung der vorliegenden Erfindung kann in den für die temporäre Umformung von Haaren üblichen Formen konfektioniert sein, z. B. als Haargel, Haarspray Haarschaum oder Haarwachs. Bevorzugt ist die Konfektionierung als Haargel.

Sowohl Haarschäume als auch Haarsprays erfordern die Anwesenheit von Treibmitteln. Erfindungsgemäß sollten dafür jedoch bevorzugt keine oder nur geringe Mengen an Kohlenwasserstoffen eingesetzt werden. Propan, Propan/Butan-Gemische und Dimethylether sind erfindungsgemäß besonders geeignete Treibmittel.

### Beispiele

Es wurde folgende Haargele hergestellt:

| **Komponente/Rohstoff** | **INCI-Bezeichnung oder chemische Bezeichnung** | **V1** | **V2** | **E1** |
|---|---|---|---|---|
| Tilamar® Fix A140 ¹ | Acrylates Copolymer | 12,5 | -- | 6,25 |
| AquaStyle SH-100 ² | Acrylates Copolymer (and) Water | -- | 16,5 | 8,25 |
| AMP-ULTRA PC 2000 | Aminomethyl Propanol | 3,0 | 0,3 | 0,3 |
| Wasser | | 84,5 | 83,2 | 85,2 |
| **Gesamt** | | 100 | 100 | 100 |

| | | | | |
|---|---|---|---|---|
| ¹ 40 Gew.-% Aktivsubstanz in Wasser ² 30 Gew.-% Aktivsubstanz in Wasser | | | | |

Die Mengenangaben in der Tabelle sind in Gew.-% des jeweiligen Rohstoffs, bezogen auf die gesamte Zusammensetzung, angegeben. Der Polymergehalt in jeder der Zusammensetzungen V1, V2 und E1 betrug 5,0 Gew.-%.

Für die erhaltenen Stylingmittel wurde mittels eines HHCR-Test (High Humidity Curl Retention-Test: 6h) an aufgereinigten Kerling-Haarsträhnen die Feuchtebeständigkeit bestimmt (Mittelwert bei Bestimmung an je 5 Haarsträhnen):

| | **V1** | **V2** | **E1** |
|---|---|---|---|
| HHCR | 67% | 72% | 86% |

Die erfindungsgemäß verwendete Polymerkombination E1 zeigte demnach einen deutlich überadditiven, synergistischen Effekt in Bezug auf die Feuchtebeständigkeit.

## Patentansprüche

1. Verwendung einer kosmetischen Zusammensetzung zur temporären Umformung keratinischer Fasern, welche enthält:
(a) mindestens ein Acrylatcopolymer (a), welches auschließlich aus folgenden Monomereinheiten aufgebaut ist:
(a1) n-Butylmethacrylat,
(a2) Methacrylsäure,
(a3) Ethylacrylat,
und
(b) mindestens ein anionisches Acrylatcopolymer (b), welches von dem Acrylatcopolymer (a) verschieden ist und nur aus den Monomereinheiten (b1), (b2) und (b3) aufgebaut ist, wobei
(b1) mindestens eine (Meth)Acrylsäureeinheit, welche Methacrylsäure ist,
(b2) mindestens eine (Meth)Acrylsäureethylestereinheit, welche Ethylacrylat ist, und
(b3) mindestens eine (Meth)Acrylsäureestereinheit, die von der (Meth)Acrylsäureethylestereinheit (b2) verschieden ist und welche ein (Meth)Acrylsäurealkylester mit einer linearen oder verzweigten Alkylgruppe mit 2 bis 12 Kohlenstoffatomen ist,
aufweist,
zur Verbesserung der Feuchtebeständigkeit temporär verformter keratinischer Fasern.

2. Verwendung nach Anspruch 1, wobei das mindestens eine Acrylatcopolymer (a) bezogen auf das Gewicht des Copolymers (a) 60 bis 80 Gew.-% n-Butylmethacrylat, 10 bis 30 Gew.-% n-Methacrylsäure und 5 bis 15 Gew.-% Ethylacrylat aufweist.

3. Verwendung nach einem der vorhergehenden Ansprüche, wobei die Zusammensetzung das Copolymer (a) in einem Anteil von 0,2 bis 4,0 Gew.-%, bevorzugt 1,0 bis 3,5 Gew.-% und insbesondere von 1,5 bis 3,0 Gew.-% enthält, bezogen auf das Gesamtgewicht der kosmetischen Zusammensetzung.

4. Verwendung nach einem der vorhergehenden Ansprüche, wobei die Zusammensetzung das anionische Acrylatcopolymer (b) in einem Anteil von 0,1 bis 5,0 Gew.-%, bevorzugt 1,0 bis 4,0 Gew.-% und insbesondere von 1,5 bis 3,0 Gew.-% enthält, bezogen auf das Gesamtgewicht der kosmetischen Zusammensetzung.

5. Verwendung nach einem der vorhergehenden Ansprüche, wobei die Zusammensetzung weiterhin mindestens ein von den Acrylatcopolymeren (a) und (b) verschiedenes Polymer (c), insbesondere ein anionisches oder nichtionisches Polymer (c), enthält.

6. Verwendung nach einem der vorhergehenden Ansprüche, wobei die Zusammensetzung als Haargel, Haarspray, Haarschaum oder Haarwachs, insbesondere als Haargel, vorliegt.

## Claims

1. The use of a cosmetic composition for temporarily reshaping keratin fibers that contains:
(a) at least one acrylate copolymer (a) which is composed exclusively of the following monomer units:
(a1) n-butyl methacrylate,
(a2) methacrylic acid,
(a3) ethyl acrylate, and
(b) at least one anionic acrylate copolymer (b) which is different from the acrylate copolymer (a) and composed only of the monomer units (b1), (b2) and (b3), wherein
(b1) at least one (meth)acrylic acid unit which is methacrylic acid,
(b2) at least one (meth)acrylic acid ethyl ester unit which is ethyl acrylate, and
(b3) at least one (meth)acrylic acid ester unit which is different from the (meth)acrylic acid ethyl ester unit (b2) and is a (meth)acrylic acid alkyl ester having a linear or branched alkyl group having 2 to 12 carbon atoms for improving the moisture resistance of temporarily deformed keratin fibers.

2. The use according to claim 1, wherein the at least one acrylate copolymer (a), based on the weight of the copolymer (a), comprises 60 to 80 wt.% of n-butyl methacrylate, 10 to 30 wt.% of n-methacrylic acid and 5 to 15 wt.% of ethyl acrylate.

3. The use according to one of the preceding claims, wherein the composition contains the copolymer (a) in a proportion of from 0.2 to 4.0 wt.%, preferably from 1.0 to 3.5 wt.%, and in particular from 1.5 to 3.0 wt.%, based on the total weight of the cosmetic composition.

4. The use according to one of the preceding claims, wherein the composition contains the anionic acrylate copolymer (b) in a proportion of from 0.1 to 5.0 wt.%, preferably from 1.0 to 4.0 wt.%, and in particular from 1.5 to 3.0 wt.%, based on the total weight of the cosmetic composition.

5. The use according to one of the preceding claims, wherein the composition further contains at least one polymer (c) which is different from acrylate copolymers (a) and (b), in particular an anionic or non-ionic polymer (c).

6. The use according to one of the preceding claims, wherein the composition is a hair gel, hair spray, hair foam, or hair wax, in particular a hair gel.

## Revendications

1. Utilisation d'une composition cosmétique pour la transformation temporaire des fibres kératiniques, laquelle composition comprend :
(a) au moins un copolymère d'acrylate (a) composé exclusivement des unités monomères suivantes :
(a1) méthacrylate de n-butyle,
(a2) acide méthacrylique,
(a3) acrylate d'éthyle, et
(b) au moins un copolymère anionique d'acrylate (b) différent du copolymère d'acrylate (a) et composé uniquement des unités monomères (b1), (b2) et (b3), où
(b1) présente au moins une unité d'acide (méth)acrylique qui est l'acide méthacrylique,
(b2) présente au moins une unité ester éthylique d'acide (méth)acrylique qui est l'acrylate d'éthyle, et
(b3) au moins une unité ester d'acide (méth)acrylique différent de l'unité ester éthylique d'acide (méth)acrylique (b2) et qui est un ester alkylique d'acide (méth)acrylique ayant un groupe alkyle linéaire ou ramifié comprenant 2 à 12 atomes de carbone, pour améliorer la résistance à l'humidité des fibres kératiniques temporairement difformées.

2. Utilisation selon la revendication 1, dans laquelle l'au moins un copolymère d'acrylate (a) par rapport au poids du copolymère (a) présente 60 à 80 % en poids de méthacrylate de n-butyle, 10 à 30 % en poids d'acide n-méthacrylique et 5 à 15 % en poids d'acrylate d'éthyle.

3. Utilisation selon l'une des revendications précédentes, dans laquelle la composition contient le copolymère (a) dans une proportion de 0,2 à 4,0 % en poids, de préférence de 1,0 à 3,5 % en poids et en particulier de 1,5 à 3,0 % en poids par rapport au poids total de la composition cosmétique.

4. Utilisation selon l'une des revendications précédentes, dans laquelle la composition contient le copolymère anionique d'acrylate (b) dans une proportion de 0,1 à 5,0 % en poids, de préférence de 1,0 à 4,0 % en poids et en particulier de 1,5 à 3,0 % en poids par rapport au poids total de la composition cosmétique.

5. Utilisation selon l'une des revendications précédentes, dans laquelle la composition comprend en outre au moins un polymère (c) différent des copolymères d'acrylate (a) et (b), en particulier un polymère anionique ou non ionique (c).

6. Utilisation selon l'une des revendications précédentes, dans laquelle la composition est présente sous forme de gel capillaire, de laque, de mousse ou de cire capillaire, en particulier sous forme de gel capillaire.
